(19) Europäisches Patentamt
European Patent Office
Office européen des brevets

(11) Publication number: 0 038 600
A1

(12) **EUROPEAN PATENT APPLICATION**

(21) Application number: 81200425.7

(22) Date of filing: 15.04.81

(51) Int. Cl.³: C 07 D 307/32
C 07 C 59/52, C 07 C 59/64
C 07 C 69/017, A 61 K 31/19
A 61 K 31/365

(30) Priority: 21.04.80 GB 8013040

(43) Date of publication of application:
28.10.81 Bulletin 81/43

(84) Designated Contracting States:
AT BE CH DE FR GB IT LI LU NL SE

(71) Applicant: Akzo N.V.
IJssellaan 82
NL-6826 DW Arnhem(NL)

(72) Inventor: Stitch, Stanley Roy
26-28 Hyde Terrace
Leeds LS 2 9LN(GB)

(72) Inventor: Toumba, Jack Kyriacos
205 King's Cross Road
London WC1X 9DB(GB)

(72) Inventor: Groen, Marinus Bernard
Bossestraat 53
Schayk(NL)

(72) Inventor: Funke, Carel Wilhelmus
Peellaan 31
Oss(NL)

(74) Representative: Hermans, Franciscus G.M. et al,
Postbus 20
NL-5340 BH Oss(NL)

(54) **Bis-hydroxybenzyl derivatives, processes for their preparation and pharmaceutical composition containing same.**

(57) The invention relates to compounds of the formula's:

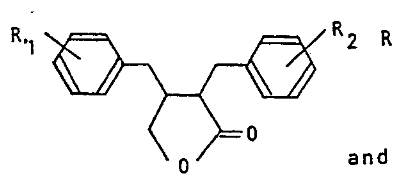

and

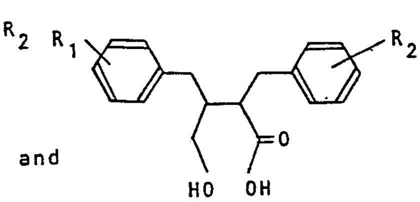

1

1A

and salts thereof, in which $R_1$ and $R_2$ may be the same or different groups selected from hydroxy, and etherified hydroxy and an esterified hydroxy group, having valuable anti-inflammatory, lipid lowering activities, and immuno-modulating properties.

EP 0 038 600 A1

Croydon Printing Company Ltd.

BIS-HYDROXYBENZYL DERIVATIVES, processes for their preparation and pharmaceutical composition containing same.

The invention relates to bis-hydroxybenzyl derivatives, to processes for their preparation and to pharmaceutical compositions containing same.

More particularly the invention relates to compounds of the formula's:

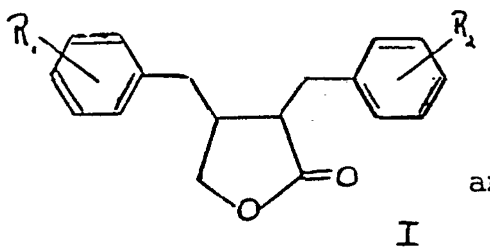

I

and

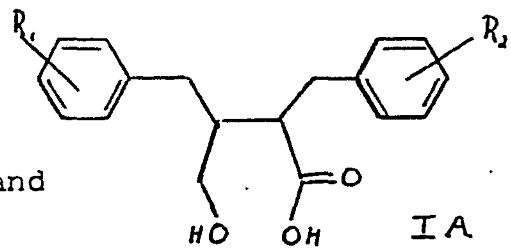

IA

and salts thereof, in which $R_1$ and $R_2$ may be the same or different groups selected from hydroxy, an etherified hydroxy and an esterified hydroxy group.

The compounds of formula's I and IA possess anti-inflammatory and lipid lowering activities and are potent immuno-modulators with low toxicity. They enhance cell mediated immunity, stimulate the action of phagocytic cells and inhibit complement. They can generate a strong cell killing activity against foreign cells, such as cancercells. They are also useful for the control of pregnancy, as they can modify tolerance of foetal tissue.

The compounds according to the invention may be prepared by methods in actual use or described in the literature.

For example, the compounds of formula I may be prepared in two steps from

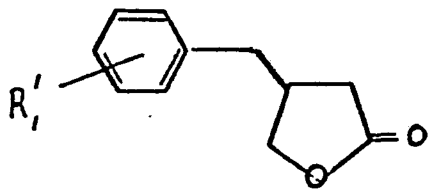

II

in which $R_1'$ is an etherified or esterified hydroxy group by reacting with a lithium compound such as lithium di-isopropylamide, followed by reacting the lithium derivative thus obtained with the compound

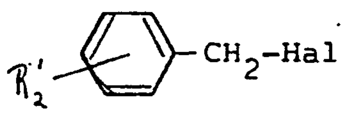

III

in which $R_2'$ is an etherified or esterified hydroxyl group and Hal represents halogen, preferably chlorine or bromine.

The free hydroxy compound of formula I ($R_1$ and $R_2$ are hydroxy) can be obtained by removing the hydroxy protecting group (the ether or ester moiety) in the usual manner.
For example, an alkyloxy group such as methoxy can be converted into the hydroxy group by the action of e.g. borontribromide, and a trimethylsilyloxy, a tetrahydropyranyloxy or an acyloxy group can be converted into the hydroxy group under (weakly) acidic conditions.

The preparation of the required starting material of formula II may be prepared as disclosed in the attached flow sheet.

Flow sheet

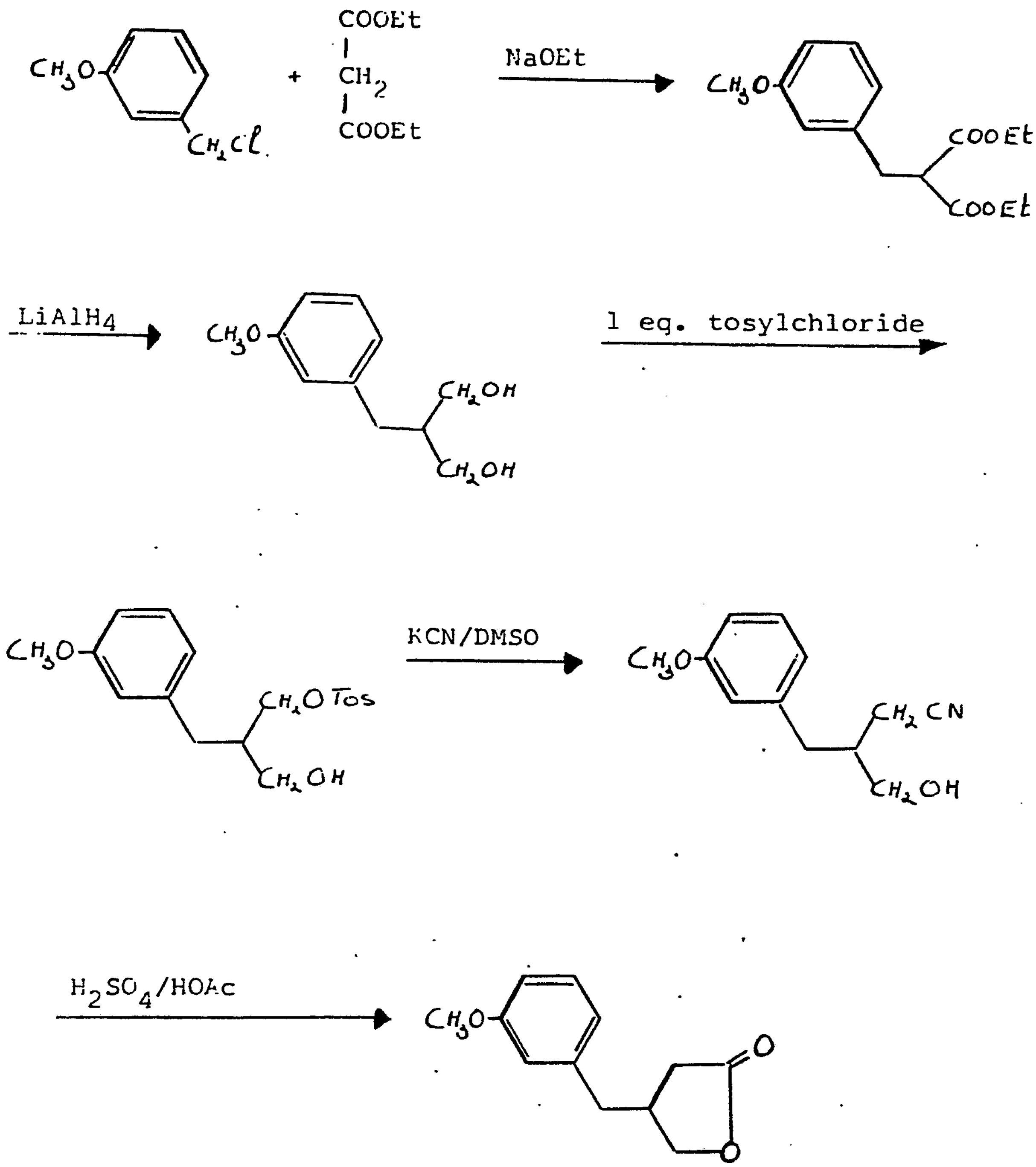

Compound II can also be used as starting product for a reaction with a substituted benzaldehyde under strong alkaline conditions, such as sodiummethoxide, sodiumamide or potassiumtert.butylate in an inert solvent, such as benzene or ether. The resulting compound of the general formula IV:

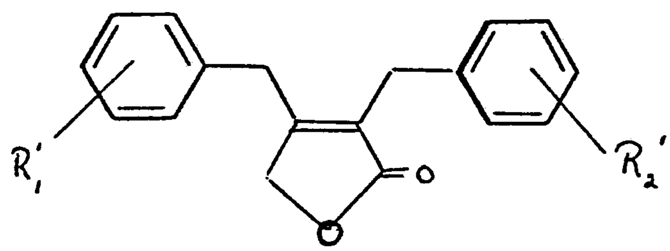

IV

in which $R_1$' and $R_2$' have the aforesaid meanings, can be converted into the final product I by reduction of the double bond in a well-known manner, preferably by catalytic hydrogenation, e.g. with palladium on carbon as catalyst.

Another convenient synthesis for the preparation of the compounds I consists of a reduction of an oxo group of a compound of the formula V:

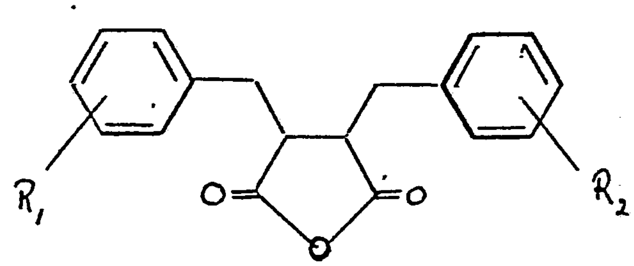

V

in which $R_1$ and $R_2$ have the aforesaid meanings.

This reduction can be carried out by conventional means, for example with aluminum-amalgam or sodium borohydride in a suitable solvent such as dimethylformamide.

The compound V can be prepared according to the following reaction-scheme:

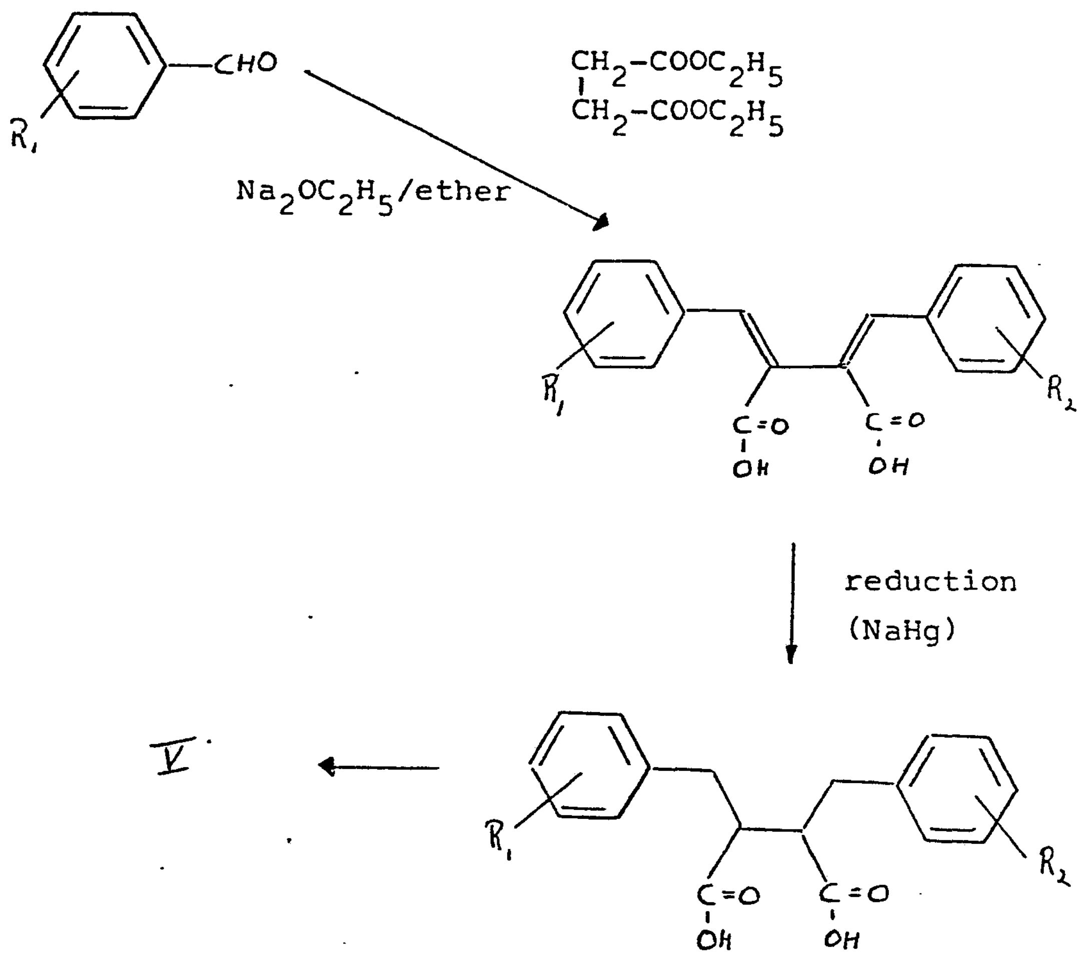

In aqueous (weakly) alkaline conditions the compounds of formula I are mainly present in "open" form, namely as compounds of the formula:

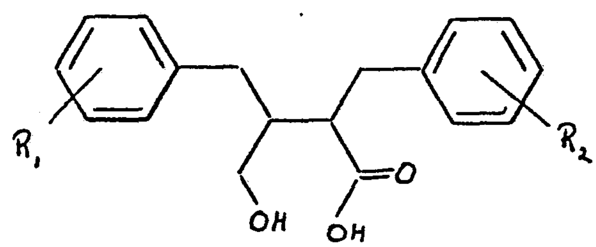

IA

in which $R_1$ and $R_2$ have the meanings assigned above.

These compounds of formula IA as well as salts thereof are also considered part of the present invention.

Obviously the compounds of formula IA can be obtained directly by using one of the aforesaid reaction methods but using aqueous alkaline conditions with respect to the isolation of the endproduct.

The salts according to this invention are usually the alkaline metal salts (pref. Na) or ammonium salts.

An etherified hydroxy group is usually a hydrocarbon-oxy radical with 1 to 6 carbon atoms, preferably 1 to 4 carbon atoms, such as methoxy, ethoxy, allyloxy, propyloxy, isopropyloxy, butoxy etc., but also other ethermoieties are possible such as a trimethylsilyloxy and tetrahydropyranyl-2-oxy group.

An esterified hydroxy group is usually an acyloxy group, in which the acyl group is derived from an aliphatic carboxylic acid with 1-6 carbon atoms, preferably 1 to 4 carbon atoms, such as acetic acid, propionic acid and butyric acid.

The compounds I and IA can occur in two diastereo-isomeric forms (cis/trans and threo/ erythro respectively). Both diastereo-isomers as well as the mixture thereof are compounds according to this invention. The aforesaid processes II ⟶ I and V ⟶ I usually result in a mixture of the cis and trans isomer, whereby the trans isomer is the major product; the process IV ⟶ I results in the cis isomer as the major product. If necessary the mixture of diastereo-isomers can be separated in the usual manner by e.g. crystallisation, column chromatography, preparative thin layer chromatography, counter current distribution, etc.

Moreover each cis and trans compound is a racemic mixture. The separate optical enantiomers also belong to the compounds of the invention. They may be isolated from the racemate in the usual manner, e.g. using an optically active base, or may be prepared starting from an optically active starting product.

The compounds according to the invention can be processed to customary liquid or solid pharmaceutical preparations, for example to sugar-coated pills, tablets, suppositories and solutions, also for injections. The customary excipients and diluents are used for this purpose.

The oral single dose ranges from 0,01-100 mg/kg body weight, the oral daily dose ranges from 0,03 to 300 mg/kg bodyweight. The parenteral single dose ranges from 1 $\gamma$ to 10 mg/kg body weight; the daily dose is about the threefold quantity of this single dose. As anti-inflammatory drug, the compound of the invention may also be incorporated in an ointment, gel or paste for local application. Preferably the drug is present in the ointment, gel or paste in an amount of 0,01 up to 2,5% of the total composition.

Compounds which are recommended are compounds of formula I, in which $R_1$ and $R_2$ both represent hydroxy groups, substituted at the benzyl moieties in meta position. More particularly the trans isomer of the formula:

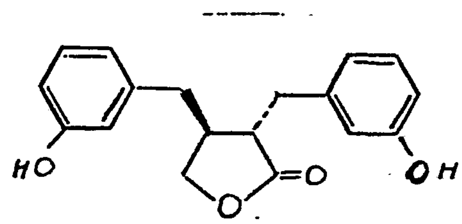

is preferred.

Example 1

trans-dihydro-3,4-bis[(3-methoxyphenyl)methyl]2(3H)furanone

A solution of lithium di-isopropylamide was prepared by stirring a mixture of 1,01 g (10 mmol) di-isopropylamine, 6,3 ml 1.6 M n-butyllithium in hexane and 10 ml dry tetrahydrofuran (THF) for 10 minutes under nitrogen atmosphere at 0 °C. The resulting solution was cooled down to -70 °C, after which 1,85 g (9 mmol) dihydro-4-[(3-methoxyphenyl)methyl]2(3H)furanone dissolved in 5 ml dry THF were added dropwise. The resulting mixture was stirred for 20 minutes at -70 °C after which a mixture of 2,01 g (10 mmol) 3-methoxybenzylbromide and 2,0 g (11 mmol) hexamethylphosphoric acid triamide was added dropwise.
The resulting mixture was stirred for 2 hours at -40 °C.

This reaction mixture is added to a solution of ammoniumchloride and subsequently extracted into ehtylacetate. The extracts were washed with water and dried on sodium sulphate; then the solvent is evaporated. The residue was chromatographed over

silica gel using the solvent system hexane/ethyl acetate (8:2).

Rf = 0,46 in hexane/ethylacetate (6:4) on $SiO_2$.

Yield: 1,97 g (67%).

## Example 2

trans-dihydro-3,4-bis[3-hydroxyphenyl)methyl]2(3H)furanone

To a solution of 1,63 g (5 mmol) dihydro-3,4-bis[(3-methoxyphenyl)mehtyl]2(3H) furanone in 10 ml dichloromethane was added dropwise 1,5 ml (16 mmol) borontribromide under nitrogen atmosphere at -78 °C. The resulting solution was slowly warmed to 0 °C while stirring. Water was added dropwise to the reaction mixture, after which the mixture was carefully extracted with ethylacetate. The extracts were washed with NaCl-solution, dried and evaporated.

The residue was crystallised from chloroform yielding 1,29 g of product; mp. 141 - 143 °C

NMR ($CDCl_3$, 200 MHz)

2.55 (m, 4)
2.96 (m, 2)
3.90 (dd, 1, J = 7 and 9, $H_5$)
4.13 (dd, 1, J = 7 and 9, $H_5'$)
6.5 - 6.8 (m, 4, aromatic protons)
7.17 (t, 1, J = 9) and
7.20 (t, 1, J = 9 aromatic protons).

Example 3

trans-(-)-dihydro-3,4-bis[3-methoxyphenyl)methyl]-2(3H)-furanone

To a solution of 4,12 g (20 mmol) of (±)-dihydro-4-[(3-methoxyphenyl)methyl]-2(3H)-furanone in 20 ml of methanol was added 40 ml of 4% aqueous KOH. The mixture was heated at 50 °C for 30 minutes, resulting in a clear solution, which was cooled to 0 - 5 °C. The reaction mixture was acidified with 2 N HCl at <5 °C and extracted with icecold ether (2x 50 ml). The extracts were dried briefly over anhydrous $Na_2SO_4$ and filtered. To the filtrate was added 2,7 g (20 mmol) of d-amphetamine and the precipitated salt was collected by filtration; yield 6,5 g, mp. 95 - 105 °C. This product was recrystallized four times from ethyl acetate to give 1,7 g of material with mp. 123 - 125 °C. This was treated with 40 ml 1N aqueous HCl at 50 °C. The cooled reactions mixture was extracted with ether (2x 50 ml) and the extracts were dried over anhydrous $Na_2SO_4$ and evaporated to give 0,94 g of (+)-dihydro-4-[(3-methoxyphenyl)methyl]-2(3H)-furanone, $[\alpha]_D^{20}$ = +6,35 ° (C1, $CHCl_3$).
This material was converted in the same manner as described in example 1 to give the desired product, yield 1,31 g (88%), $[\alpha]_D^{20}$ = -40,6 (C1, $CHCl_3$).

Example 4

trans-(-)-dihydro-3,4-bis[(3-hydroxyphenyl)methyl]-2(3H)-furanone

The product of example 3 was treated in the same manner as described in example 2 to give the desired product in 69% yield, mp. 125-130 °C, $[\alpha]_D^{20}$ = -38,4 ° (C 0,5, $CHCl_3$).

## Example 5

cis-dihydro-3,4-bis[(3-methoxyphenyl)methyl]-2(3H)-furanone.

A mixture of 2,06 g (10 mmol) of dihydro-4-[(3-methoxy-phenyl)methyl]-2(3H)-furanone, 1.36 g (10 mmol) of 3-methoxy benzaldehyde, 0.54 g (10 mmol) of sodium methoxide and 20 ml of benzene was stirred for 24h at room temperature. The reactionmixture was washed with water, dried over anhydrous $Na_2SO_4$ and concentrated. The residue was chromatographed over silica gel with 7:3 hexane /ethylacetate to give 1.19 g (37%) of (E)-dihydro-4-[(3-methoxyphenyl)methyl)]-3-[(3-methoxyphenyl)methylene] 2(3H)-furanone. This product was dissolved in 200 ml of ethylacetate mixed with o.69 g of 5% paladium on carbon catalyst and hydrogenated at atmospheric pressure for 2 hours. Filtration and evaporation of the solvent gave the desired product in quantitative yield. Rf= o.48 in hexane/ethylacetate 6:4 on $SiO_2$

## Example 6

cis-dihydro-3,4-bis[(3-hydroxyphenyl)methyl]-2(3H)-furanone

The product of example 5 was treated in the same manner as described in example 2 to give the desired product in 70% yield, m.p. 157-158 $^oC$.

## Example 7

trans-3,4-bis[(3-acetoxyphenyl)methyl]dihydro-2(3H)-furanone

The product of example 2 (1,49 g, 5 mmol) was mixed with 7,5 ml of pyridine, 7,5 ml of acetic anhydride and 45 ml of dry dichloromethane.

The mixture was kept at room temperature for 2 hours and then diluted with water. The organic layer was separated, washed several times with water and aqueous sodium bicarbonate and dried over anhydrous $K_2CO_3$. The solvent was evaporated and the residue was chromatographed over Silica gel with hexane/ethyl acetate (1:1) In this manner 1,76 g (92% yield) of product was obtained as a colourless oil.

Rf = 0.30 in hexane/ethyl acetate 6 : 4 on $SiO_2$.

## Example 8

In a similar manner were obtained:

trans-(-)-dihydro-3,4-bis[(3-acetoxyphenyl)methyl] 2(3H)-furanone

cis-dihydro-3,4-bis[(3-acetoxyphenyl)methyl] 2(3H)-furanone

## Example 9

trans-dihydro-3,4-bis[(3-hydroxyphenyl)methyl]-2(3H)-furanone monohemisuccinate

The product of example 2 (0,90, 3 mmol) was mixed with 0,4 g (4 mmol) of succinic anhydride and 3 ml of pyridine. The mixture was stirred for 24 hours at room temperature, diluted with water and extracted with ethyl acetate. The extracts were washed with aqueous HCl, dried over anhydrous $Na_2SO_4$ and concentrated *in vacuo*. The residu was chromatographed over silica gel (30 g) with hexane/acetone 1:1. First 0,27 g (30%) of starting material was eluted, followed by 0,6 g (50%) of product (viscous oil).

Rf = 0,36 in hexane/acetone/acetic acid (1:1:0,015) on $SiO_2$.

## Example 10

sodium salt of 3-hydroxymethyl-4-(3-methoxyphenyl)-2-(3-methoxyphenylmethyl) butyric acid. (formula IA)

0,3 g of the compound obtained in example 1 was mixed with 2 ml of 1 N aqueous sodiumhydroxide. The mixture was heated at 50 °C for 30 minutes and then concentrated in vacuo, resulting in 0,35 g of amorphous solid.

Claims

1. Compound of the formula

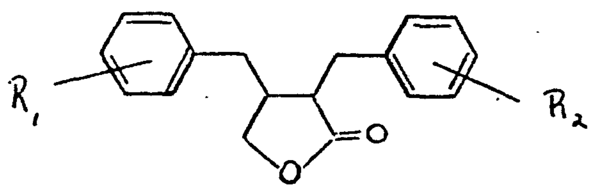

or

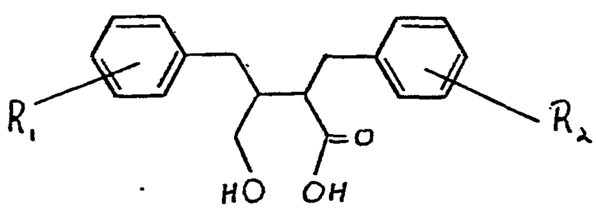

and salts thereof,
in which $R_1$ and $R_2$ are the same or different groups selected from hydroxy, an etherified hydroxy and an esterified hydroxy group.

2. A compound according to claim 1 of the formula:

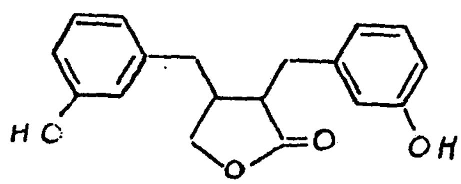

3. A compound according to claim 1 of the formula:

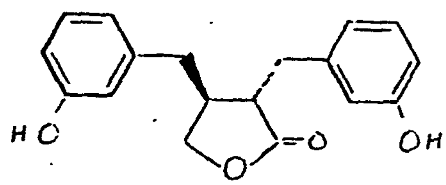

4. Process for the preparation of the compounds indicated in claim 1, characterized in that they are prepared by methods in actual use or described in the literature.

5. Pharmaceutical composition containing a compound according to claim 1 and a pharmaceutically acceptable carrier or diluent.

0038600

European Patent Office

Application number

# EUROPEAN SEARCH REPORT

EP 81200425.7

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| X | DE - A1 - 2 657 346 (NATIONAL DISTILLERS) (02-07-1980)<br>+ Totality; especially claim 1; page 8, lines 12-25; page 9, lines 13-25 +<br>& GB-A-1 570 600 (02-07-1980)<br>---- | 1-4 | C 07 D 307/32<br>C 07 D 59/52<br>C 07 C 59/64<br>C 07 C 69/017<br>A 61 K 31/19<br>A 61 K 31/365 |
| | | | TECHNICAL FIELDS SEARCHED (Int. Cl.3) |
| | | | C 07 D 307/00<br>C 07 C 59/00<br>C 07 C 69/00<br>A 61 K 31/00 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 21-07-1981 | ONDER |

EPO Form 1503.1 06.78